# EUROPEAN PATENT APPLICATION

(11) **EP 2 436 302 A1**
(43) Date of publication of application: **04.04.2012**
(21) Application number: 11007656.9
(22) Date of filing: 20.09.2011
(51) Int. Cl.: A61B 1/06

(54) **Illumination apparatus and examination system**

(30) Priority: 30.09.2010 JP 2010222668
(71) Applicant: OLYMPUS CORPORATION, Shibuya-ku Tokyo 151-0072 (JP)
(72) Inventor: Kumai, Katsunori, Shibuya-ku, Tokyo 151-0072 (JP)
(74) Representative: Gunzelmann, Rainer

(57) **Abstract**

Provided is an illumination apparatus that can switch between at least two illumination modes having different spectral characteristics in a short period of time.

The illumination apparatus includes light sources 1 and 2 configured to generate light in wavelength bands required for at least two illumination modes; a dichroic mirror 5 configured to transmit light in a predetermined band from the light source 1, to transmit light in the predetermined band from the light source 2 while reflecting light that is outside the predetermined band in the direction of the optical axis of light from the light source 1, and to combine the light from the light source 1 and the light from the light source 2; and a control unit configured to control the illumination states of the light sources 1 and 2.

## Description

### {Technical Field}

The present invention relates to an illumination apparatus and an examination system including the same.

### {Background Art}

There is a known illumination apparatus that is provided with a movable filter, which is constituted of a filter having at least two different spectral characteristics, interposed between a white light source and an illuminated area and that moves the filter depending on an illumination mode to mechanically switch the light to be transmitted through the illuminated part, from the white light (for example, refer to PTL 1).
Another known illumination apparatus has a brightness priority mode and color priority mode and outputs a mode setting signal to electrically switch between the brightness priority mode and the color priority mode (for example, refer to PTL 2).
Another known illumination apparatus partially replaces main illumination light from a lamp, etc., which is substantially white, with sub-illumination light from a laser light source, etc. and enhances the emission spectrum of the main illumination light with the sub-illumination light to generates illumination light (for example, see PTL 3).

### {Citation List}

### {Patent Literature}

{PTL 1}
   Japanese Unexamined Patent Application, Publication No. 2001-314370
{PTL 2}
   Japanese Unexamined Patent Application, Publication No. 2006-349731
{PTL 3}
   Japanese Unexamined Patent Application, Publication No. 2002-296680

### {Summary of Invention}

### {Technical Problem}

When examination or measurement of a subject is to be carried out in at least two illumination modes with different spectral characteristics, a reduction in the switching time is required for reducing the time required for the operation. Moreover, during examination, it is required that examination in multiple illumination modes be repeated in a short amount of time to make it easier to confirm the position of particular sites by displaying two screens on a video monitor or by overlaying two screens. Specifically, an example of endoscopy includes fluoroscopy, etc. that identifies the position of an affected area by overlaying the affected area, which emits fluorescence of a predetermined wavelength, on an examination image obtained with white illumination light.

Unfortunately, with the switching method disclosed in PTL 1, because the filters are mechanically moved, it takes some time to switch the filters, and thus, the time required for the operation increase. Moreover, if the boundary regions of different filters are disposed in the optical path during switching, the problem of image distortion occurs.

With the switching method disclosed in PTL 2, the driving conditions of an LED can be changed depending on the illumination mode to perform instantaneous mode switching. However, one problem is that the desired spectral characteristic often cannot be acquired because only the color balance of the illumination apparatus, which is constituted of individual RGB light sources, can be changed.

PTL 3 discloses an illumination apparatus in which there is illumination-light combining means for enhancing a predetermined wavelength band of an emission spectrum of main illumination light with sub-illumination light and generating illumination light by replacing the main illumination with the sub-illumination light in a predetermined wavelength band in which the light intensity of the main illumination light is smaller than that of the sub-illumination light from a light source; however, there is no disclosure associated with the switching of illumination modes, and thus, inconveniently, in some cases, the desired spectral characteristic cannot be acquired.

The present invention has been conceived in light of the circumstances described above, and an object thereof is to provide an illumination apparatus and an examination system that can switch between at least two illumination modes having different spectral characteristics.

### {Solution to Problem}

To achieve the object described above, the present invention provides the following solutions.
A first aspect of the present invention is an illumination apparatus configured to illuminate an illumination area in a plurality of illumination modes constituted of light in different predetermined bands, having bands in which a first band required by one illumination mode of the plurality of illumination modes is narrower than a second band required by another illumination mode, the apparatus including a plurality of light sources configured to emit illumination light in at least the first band; a light combining unit configured to transmit light in the first band, among the illumination light emitted from at least one light source of the plurality of light sources, toward the illumination area as transmitted light while reflecting light that is outside the first band, among the illumination light emitted from the other light sources, toward the illumination area as reflected light, and to optically combine the transmitted light and the reflected light; an illumination-mode selecting unit configured to select one illumination mode from the plurality of illumination modes; and a control unit configured to control the light intensity of light emitted from the plurality of light sources on the basis of the selected illumination mode, wherein the one illumination mode is a first mode for illuminating only the one light source, and the other mode is a second illumination mode for illuminating both the one light source and the other light sources.

A second aspect, which is described below and differs from the first aspect in that the positions of the light sources with respect to the light combining unit differ, may be employed.
A second aspect of the present invention is an illumination apparatus configured to illuminate an illumination area in a plurality of illumination modes constituted of light in different predetermined bands, having bands in which a first band required by one illumination mode of the plurality of illumination modes is narrower than a second band required by another illumination mode, the apparatus including a plurality of light sources configured to emit illumination light in at least the first band; a light combining unit configured to reflect light in the first band, among the illumination light emitted from at least one light source of the plurality of light sources, toward the illumination area as reflected light while transmitting light that is outside the first band, among the illumination light emitted from the other light sources, toward the illumination area as transmitted light, and to optically combine the transmitted light and the reflected light; an illumination-mode selecting unit configured to select one illumination mode from the plurality of illumination modes; and a control unit configured to control the light intensity of light emitted from the plurality of light sources on the basis of the selected illumination mode, wherein the one illumination mode is a first mode for illuminating only the one light source, and the other illumination mode is a second illumination mode for illuminating both the one light source and the other light sources.

According to the first and second aspects of the present invention, illumination light in a wavelength band corresponding to the illumination mode can be provided by controlling the light intensity of the light emitted from the plurality of light sources in accordance with the illumination mode and optically combining the light emitted from the plurality of light sources.

Here, one light source and the other light sources generate light in wavelength bands that at least partially overlap. Therefore, when the one light source and the other light sources are both illuminated, the light in wavelength bands that complement each other is emitted as combined light. Moreover, when one of the one light source and the other light sources is illuminated, part of the light from the light source is emitted as combined light.

Accordingly, for example, by illuminating all light sources in a second illumination mode, regular examination can be performed by irradiating a subject with light in a wide band (for example, white light). Moreover, for example, by illuminating one of the light sources in a first illumination mode, special examination can be performed by irradiating the subject with light in a narrow band (for example, blue light). In this way, by controlling the illumination state of a plurality of light sources, the examination mode can be switched instantaneously.

Furthermore, the light combining unit is not only operated as light combining means for simply combining light from a plurality of light sources but, in the special light mode, can also be operated as a wavelength cut filter that produces a spectral characteristic with the required narrowband light, i.e. that removes components in an unwanted wavelength band. In this way, the size and cost of the apparatus can be reduced without the need to provide both the light combining means and the wavelength cut filter.

In the first and second aspects described above, the one light source and the other light sources may be light sources emitting white illumination light. In this way, light source devices of the same type (model) can be used as the plurality of light sources.

In this way, for example, by lighting the first and second light sources, regular examination can be performed by irradiating a subject with light in a wide band (for example, white light). Moreover, for example, by illuminating only the second light source, special examination can be performed by irradiating the subject with light in a narrow band (for example, blue light).

A third aspect of the present invention is an examination system including one of the illumination apparatuses described above; an irradiation optical system configured to irradiate a subject with light combined at the light combining unit; and a light receiving unit configured to receive light reflected at the subject, wherein the control unit controls the emission light intensity of the plurality of light sources on the basis of the intensity of the reflected light received by the light receiving unit.

According to the third aspect of the present invention, the subject is irradiated with the light combined at the light combining unit by the irradiation optical system, and the light reflected at the subject is received by the light receiving unit. Then, the control unit controls the emission light intensity of the plurality of light sources on the basis of the intensity of the reflected light received by the light receiving unit. In this way, the intensity of the light emitted toward the subject can be adjusted in response to the intensity of the light reflected at the subject, and the examination precision of the subject can be improved.

In the third aspect described above, the light receiving unit may be an image acquisition element configured to acquire an image of the subject, and the control unit may control the emission light intensity of the plurality of light sources so that the brightness of the image acquired by the image acquisition element is substantially constant.
In this way, an image of the subject can be acquired by the image acquisition element, the emission light intensity of the plurality of light sources can be controlled such that the brightness of the image is constant, and the subject can be examined at a constant brightness.

### {Advantageous Effects of Invention}

The present invention is advantageous in that at least two illumination modes having different spectral characteristics can be switched between in a short amount of time.

### {Brief Description of Drawings}

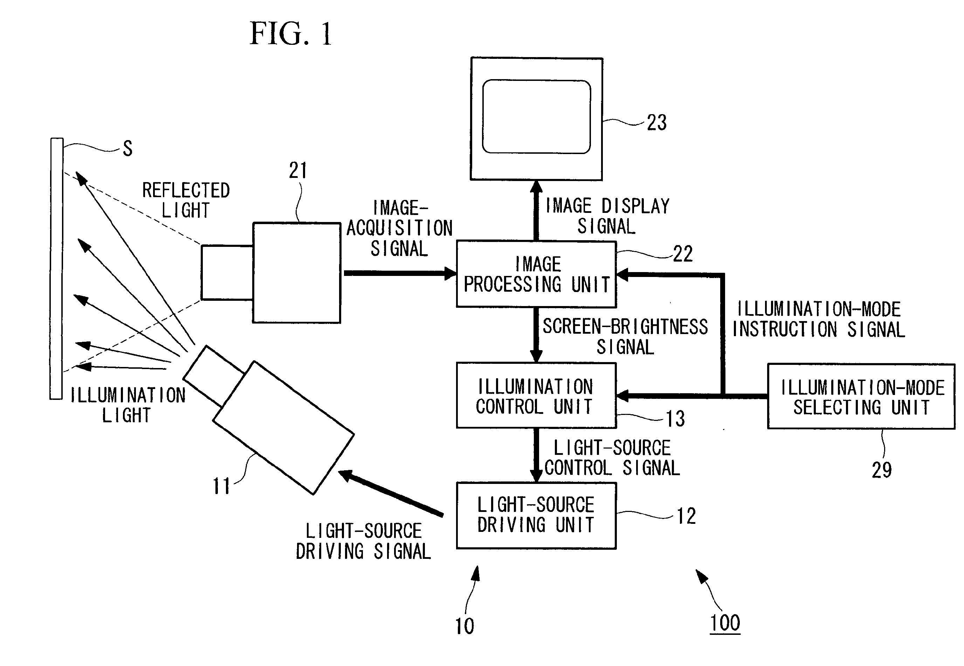

{Fig. 1}
   Fig. 1 is a schematic configuration diagram of an examination system according to embodiments of the present invention.
{Fig. 2}
   Fig. 2 is a schematic configuration diagram of an illumination apparatus according to a first embodiment of the present invention.
{Fig. 3}
   Fig. 3 is a graph illustrating the spectral characteristic of a first light source in Fig. 2.
{Fig. 4}
   Fig. 4 is a graph illustrating the spectral characteristic of a second light source in Fig. 2.
{Fig. 5}
   Fig. 5 is a graph illustrating the reflection characteristic of a dichroic mirror in Fig. 2.
{Fig. 6}
   Fig. 6 is a graph illustrating the spectral characteristic of light from the first light source in Fig. 2 after passing through the dichroic mirror.
{Fig. 7}
   Fig. 7 is a graph illustrating the spectral characteristic of light from the second light source in Fig. 2 after being reflected at the dichroic mirror.
{Fig. 8}
   Fig. 8 is a graph illustrating the spectral characteristic of illumination light (combined light obtained by combining the light in Fig. 6 and the light in Fig. 7) emitted from the illumination apparatus in Fig. 2 in a white light mode.
{Fig. 9}
   Fig. 9 is a graph illustrating the spectral characteristic of light from the second light source in Fig. 2 after being reflected at the dichroic mirror.
{Fig. 10}
   Fig. 10 is a graph illustrating the spectral characteristic of illumination light (light in Fig. 9) emitted from the illumination apparatus in Fig. 2 in a special light mode.
{Fig. 11}
   Fig. 11 is a timing chart for the illumination apparatus in Fig. 2 when the illumination mode is simply switched.
{Fig. 12}
   Fig. 12 is a timing chart for the illumination apparatus in Fig. 2 when two illumination modes are alternated in short periods of time.
{Fig. 13}
   Fig. 13 is a graph illustrating the spectral characteristic of a first light source in an illumination apparatus according to a second embodiment of the present invention.
{Fig. 14}
   Fig. 14 is a graph illustrating the spectral characteristic of a second light source in the illumination apparatus according to the second embodiment of the present invention.
{Fig. 15}
   Fig. 15 is a graph illustrating the spectral characteristic of light from the first light source in Fig. 13 after passing through a dichroic mirror.
{Fig. 16}
   Fig. 16 is a graph illustrating the spectral characteristic of light from the second light source in Fig. 14 after being reflected at the dichroic mirror.
{Fig. 17}
   Fig. 17 is a graph illustrating the spectral characteristic of illumination light (combined light obtained by combining the light in Fig. 15 and the light in Fig. 16) in a white light mode.
{Fig. 18}
   Fig. 18 is graph illustrating the spectral characteristic of illumination light (light in Fig. 16) in a special light mode.
{Fig. 19}
   Fig. 19 is a schematic configuration diagram of an illumination apparatus according to a third embodiment of the present invention.
{Fig. 20}
   Fig. 20 is a graph illustrating the reflection characteristic of a first dichroic mirror in Fig. 19.
{Fig. 21}
   Fig. 21 is a graph illustrating the reflection characteristic of a second dichroic mirror in Fig. 19.
{Fig. 22}
   Fig. 22 is a graph illustrating the spectral characteristic of light from a first light source in Fig. 19 after passing through the first dichroic mirror.
{Fig. 23}
   Fig. 23 is a graph illustrating the spectral characteristic of light from a second light source in Fig. 19 after being reflected at the first dichroic mirror.
{Fig. 24}
   Fig. 24 is a graph illustrating the spectral characteristic of light from a third light source in Fig. 19 after being reflected at the second dichroic mirror.
{Fig. 25}
   Fig. 25 is a graph illustrating the spectral characteristic of a first light source in an illumination apparatus according to a fourth embodiment of the present invention.
{Fig. 26}
   Fig. 26 is a graph illustrating the spectral characteristic of a second light source in the illumination apparatus according to the fourth embodiment of the present invention.
{Fig. 27}
   Fig. 27 is a graph illustrating the spectral characteristic of a third light source in the illumination apparatus according to the fourth embodiment of the present invention.
{Fig. 28}
   Fig. 28 is a graph illustrating the spectral characteristic of light from the first light source in Fig. 25 after passing through a first dichroic mirror.
{Fig. 29}
   Fig. 29 is a graph illustrating the spectral characteristic of light from the second light source in Fig. 26 after being reflected at the first dichroic mirror.
{Fig. 30}
   Fig. 30 is a graph illustrating the spectral characteristic of light from the third light source in Fig. 27 after being reflected at the second dichroic mirror.
{Fig. 31}
   Fig. 31 is a schematic configuration diagram of an illumination apparatus according to a fifth embodiment of the present invention.
{Fig. 32}
   Fig. 32 is a graph illustrating the reflection characteristic of a first dichroic mirror in Fig. 31.
{Fig. 33}
   Fig. 33 is a graph illustrating the reflection characteristic of a second dichroic mirror in Fig. 31.
{Fig. 34}
   Fig. 34 is a graph illustrating the spectral characteristics of first and second light sources in Fig. 31.
{Fig. 35}
   Fig. 35 is a graph illustrating the spectral characteristic of light travelling along an optical path A in the illumination apparatus in Fig. 31.
{Fig. 36}
   Fig. 36 is a graph illustrating the spectral characteristic of light travelling along an optical path B in the illumination apparatus in Fig. 31.
{Fig. 37}
   Fig. 37 is a graph illustrating the spectral characteristic of light travelling along an optical path C in the illumination apparatus in Fig. 31.
{Fig. 38}
   Fig. 38 is a longitudinal sectional diagram illustrating the schematic configuration of an illumination apparatus according to a sixth embodiment of the present invention.
{Fig. 39}
   Fig. 39 is a longitudinal sectional diagram illustrating the schematic configuration of an illumination apparatus according to a seventh embodiment of the present invention.
{Fig. 40}
   Fig. 40 is a plan view showing, in outline, the configuration of the illumination apparatus in Fig. 39.
{Fig. 41}
   Fig. 41 is a partially enlarged view of the illumination apparatus in Fig. 39.
{Fig. 42}
   Fig. 42 is a graph illustrating the spectral characteristic of a blue LED in Fig. 41.
{Fig. 43}
   Fig. 43 is a graph illustrating the spectral characteristic of a green fluorescer in Fig. 41.
{Fig. 44}
   Fig. 44 is a graph illustrating the reflection characteristic of a first dichroic prism in Fig. 41.
{Fig. 45}
   Fig. 45 is graph illustrating the spectral characteristic of a light source unit in Fig. 41.
{Fig. 46}
   Fig. 46 is a graph illustrating the reflection characteristic of a second dichroic prism in Fig. 41.
{Fig. 47}
   Fig. 47 is a graph illustrating the reflection characteristic of illumination light emitted from the illumination apparatus in Fig. 39 in a white light mode.
{Fig. 48}
   Fig. 48 is a graph illustrating the spectral characteristic of illumination light emitted from the illumination apparatus in Fig. 39 in a special light mode.

### {Description of Embodiments}

### First Embodiment

An illumination apparatus according to a first embodiment of the present invention will be described below with reference to the drawings.
Fig. 1 is a configuration diagram illustrating, in outline, an examination system 100 including an illumination apparatus according to this embodiment.
As illustrated in Fig. 1, the examination system 100 according to this embodiment includes an illumination apparatus 10 constituted of a light source unit (irradiation optical system) 11, a light-source driving unit 12, and an illumination control unit (control unit) 13; an image acquisition unit (light receiving unit) 21; an image processing unit 22; an image monitor 23; and an illumination-mode selecting unit 29.

The light source unit 11 has a plurality of light sources and emits illumination light onto a subject S. The detailed configuration of the light source unit 11 will be described below.
The image acquisition unit 21 is, for example, an image acquisition element, such as a CCD, and detects light reflected at the subject S. That is, the image acquisition unit 21 functions as light-receiving means for receiving light reflected at the subject S. Moreover, the image acquisition unit 21 acquires an image of the subject S by detecting the light reflected at the subject S and outputting an image acquisition signal to the image processing unit 22.

The image processing unit 22 processes the image acquisition signal output from the image acquisition unit 21 to generate an image of the subject S. The image processing unit 22 selects the optimal image processing for each illumination mode in response to the content of an input illumination-mode signal to generate an image signal. Additionally, the image processing unit 22 outputs the generated image of the subject S to the image monitor 23 as an image display signal and outputs the brightness of the image of the subject S to the illumination control unit 13 as a screen brightness signal.
The image monitor 23 displays the image of the subject S on a monitor screen on the basis of the image display signal output from the image processing unit 22.

The illumination control unit 13 receives a screen brightness signal output from the image processing unit 22, as well as an illumination-mode instruction signal output from the illumination-mode selecting unit 29, such as, for example, a switch. Here, the illumination-mode instruction signal is a signal that instructs the switching of illumination light to be emitted from the light source unit 11 toward the subject S to, for example, white light or special light.

The illumination-mode instruction signal is also sent to the image processing unit 22, where an image may be generated by switching to an image processing method optimal for each illumination mode. For example, in an examination method of forming a clear image of blood vessels by irradiating them with only light near 415 nm and near 540 nm, which are easily absorbed by hemoglobin in blood, reflected light near 415 nm is generated as B (blue) and G (green) channel signals of the displayed image, and reflected light near 540 nm is generated as an R (red) channel signal of the displayed image, and these signals are output to the image monitor 23.

The illumination control unit 13 generates a light-source control signal on the basis of the screen brightness signal output from the image processing unit 22 and outputs this signal to the light-source driving unit 12. Specifically, the illumination control unit 13 calculates the light intensity of the illumination light emitted from the light source unit 11 such that the brightness of the image of the subject S generated by the image processing unit 22 is constant and outputs this to the light-source driving unit 12 as a light-source control signal. Moreover, in response to the selected illumination mode, the illumination control unit 13 outputs to the light-source driving unit 12 a light-source control signal indicating which light source, among the multiple light sources in the light source unit 11, is to be lit.

When the brightness of the subject S cannot be set constant by controlling it with the light-source driving unit 12, the display gain may be set at the image processing unit 22 such that the brightness of the image of the subject S becomes constant when the image of the subject S is displayed on the image monitor 23.

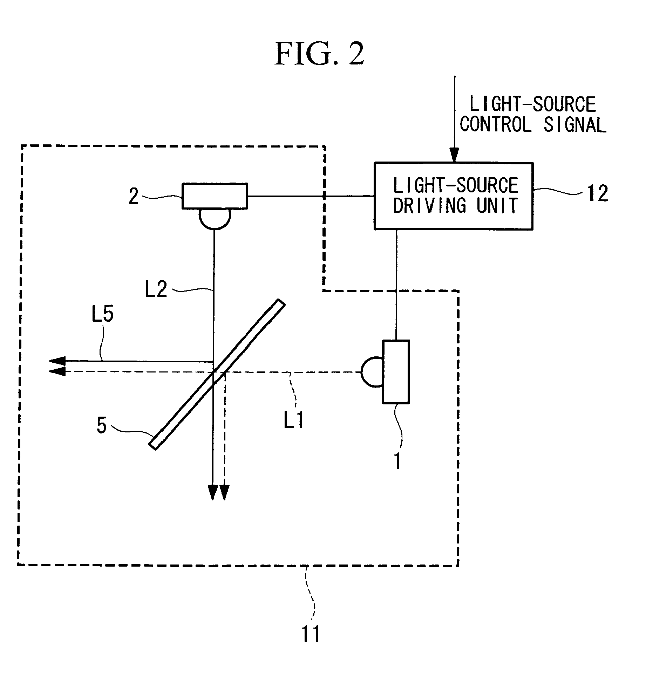

The light-source driving unit 12 drives multiple light sources in the light source unit 11 on the basis of the light-source control signal output from the illumination control unit 13.
As illustrated in Fig. 2, the light source unit 11 includes a light source (first light source) 1 and a light source (second light source) 2, which are disposed such that optical axes thereof are orthogonal to each other, and a dichroic mirror (light combining unit) 5 disposed at the intersection of the optical axis of the light source 1 and the optical axis of the light source 2.

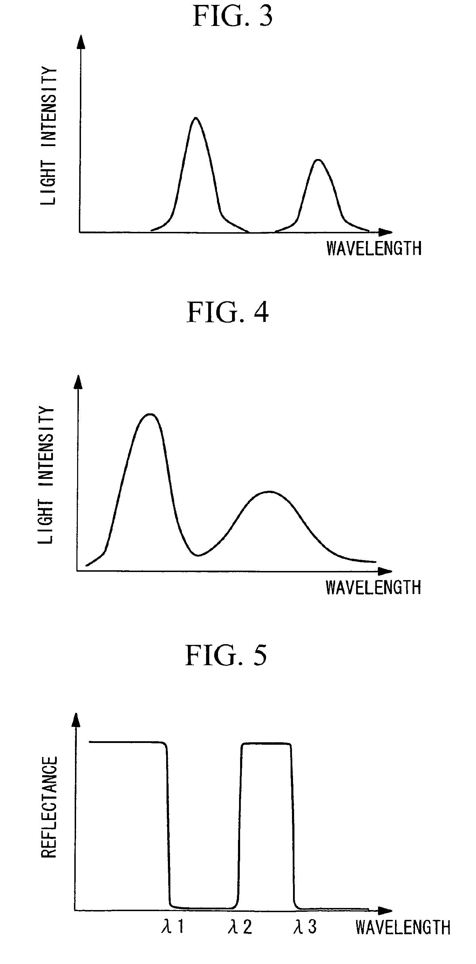

The light source 1 is a light source emitting light L1 and, as illustrated in Fig. 3, has two wavelength bands in which the light intensity peaks.
The light source 2 is a light source emitting pseudo-white light L2 and emits light such as that illustrated in Fig. 4.

As illustrated in Fig. 5, the dichroic mirror 5 has a reflection characteristic in which light in the wavelength bands between wavelengths λ1 and λ2 and greater than or equal to wavelength λ3 is transmitted, while light having a wavelength smaller than wavelength λ1 and between wavelengths λ2 and λ3 is reflected.

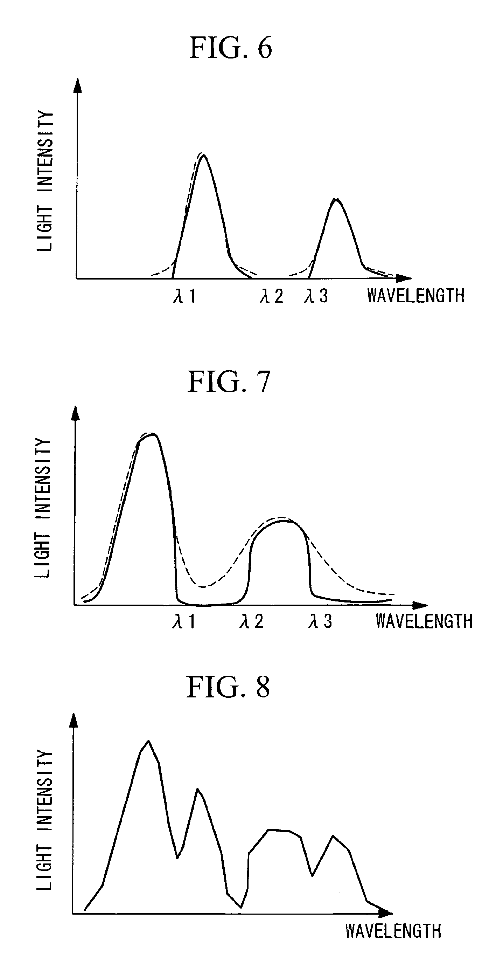

By having such a reflection characteristic, as illustrated in Fig. 6, the dichroic mirror 5 transmits light in the wavelength bands between wavelengths λ1 and λ2 and greater than or equal to wavelength λ3, among the light L1 emitted from the light source 1. Meanwhile, the dichroic mirror 5 reflects light having a wavelength smaller than wavelength λ1 and between wavelengths λ2 and λ3, among the light L1 emitted from the light source 1. In Fig. 6, the dotted line represents the light L1 emitted from the light source 1, whereas the solid line represents light transmitted through the dichroic mirror 5.

Moreover, the dichroic mirror 5 transmits light in the wavelength bands between wavelengths λ1 and λ2 and greater than or equal to wavelength λ3, among the light L2 emitted from the light source 2. Meanwhile, as illustrated in Fig. 7, the dichroic mirror 5 reflects, in the direction of the optical axis of the light L1 emitted from the light source 1, light having a wavelength smaller than wavelength λ1 and between wavelengths λ2 and λ3, among the light L2 emitted from the light source 2. In Fig. 7, the dotted line represents the light L2 emitted from the light source 2, whereas the solid line represents light reflected at the dichroic mirror 5.

Now, in a white light mode in which the subject S is irradiated with white light, the light source 1 and the light source 2 are lit.
In such a case, the dichroic mirror 5 combines the light in the wavelength bands between wavelengths λ1 and λ2 and greater than or equal to wavelength λ3, among the light L1 emitted from the light source 1, and the light below wavelength λ1 and between wavelengths λ2 and λ3, among the light L2 emitted from the light source 2 and emits this combined light L5 in the direction of the optical axis of the light L1. As illustrated in Fig. 8, the combined light L5 has a spectral characteristic resulting from superimposing light with the spectral characteristic illustrated in Fig. 6 (the light that is transmitted through the dichroic mirror 5 among the light L1 emitted from the light source 1) and light with the spectral characteristic illustrated in Fig. 7 (the light reflected at the dichroic mirror 5 among the light source L2 emitted from the light source 2).

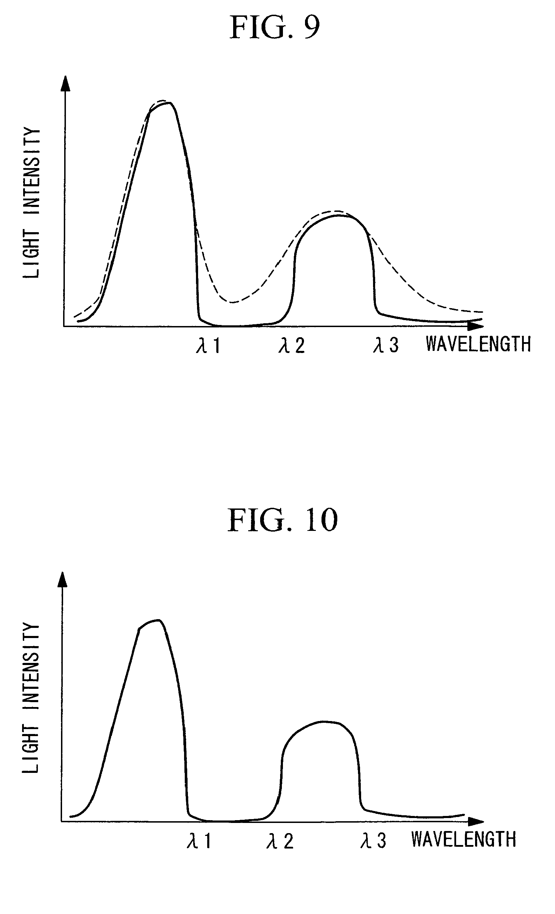

In a special light mode in which the subject S is irradiated with special light, for example, the light source 1 is turned off and only the light source 2 is lit.
In such a case, as illustrated in Fig. 9, the dichroic mirror 5 transmits light in wavelength bands between wavelengths λ1 and λ2 and greater than or equal to wavelength λ3, among the light L2 emitted from the light source 2, while reflecting and emitting light having a wavelength smaller than wavelength λ1 and between wavelengths λ2 and λ3 in the direction of the optical axis of the light L1 emitted from the light source 1. In such a case, the spectral characteristic of the light emitted in the direction of the optical axis of the light L1 is as illustrated in Fig. 10. In Fig. 9, the dotted line represents the light L2 emitted from the light source 2, whereas the solid line represents light reflected at the dichroic mirror 5.

The operation of the examination system 100 having the above-described configuration will be described below.
First, for example, a user selects one illumination mode from a plurality of illumination modes, and an illumination-mode instruction signal for driving the illumination apparatus 10 in the selected illumination mode is sent from the illumination-mode selecting unit 29.

When the selected illumination mode is the white light mode, the light source 1 and the light source 2 are both driven. In such a case, the dichroic mirror 5 combines the light in the wavelength bands between wavelengths λ1 and λ2 and greater than or equal to wavelength λ3, among the light L1 emitted from the light source 1, and the light lower than wavelength λ1 and between wavelengths λ2 and λ3, among the light L2 emitted from the light source 2, and emits this combined light L5 in the direction of the optical axis of the light L1. As illustrated in Fig. 8, the combined light L5 is white light having a wide wavelength band obtained by superimposing the spectral characteristic of the light illustrated in Fig. 6 and the spectral characteristic of the light illustrated in Fig. 7.

Alternatively, when the selected illumination mode is a special light mode, the light source 1 is turned off, and only the light source 2 is driven. In such a case, the dichroic mirror 5 emits, in the direction of the optical axis of the light L1 emitted from the light source 1, light having a wavelength smaller than wavelength λ1 and between wavelengths λ2 and λ3, among the light L2 emitted from the light source 2. In such a case, as illustrated in Fig. 10, the light emitted in the direction of the optical axis of the light L1 is special light having a narrow wavelength band.

In either illumination mode described above, the light intensity of the illumination light emitted from the light source unit 11 is controlled by the illumination control unit 13 such that the brightness of the image of the subject S is constant. Such control of the light intensity of the illumination light will be described below.
First, the subject S is irradiated with illumination light from the light source unit 11, and the light reflected at the subject S is detected by the image acquisition unit 21.

Next, the image processing unit 22 processes an image acquisition signal output from the image acquisition unit 21 to generate an image of the subject S, and this image of the subject S is displayed on the image monitor 23. Also, the brightness of the image of the subject S is output to the illumination control unit 13 as a screen brightness signal.

At the illumination control unit 13, the light intensity of the illumination light emitted from the light source unit 11 is controlled on the basis of the screen brightness signal from the image processing unit 22 such that the brightness of the image of the subject S is constant. Two cases of intensity control of the illumination light, which is mentioned above, for simply switching the illumination mode and for alternating between two illumination modes in short periods of time will be described below.

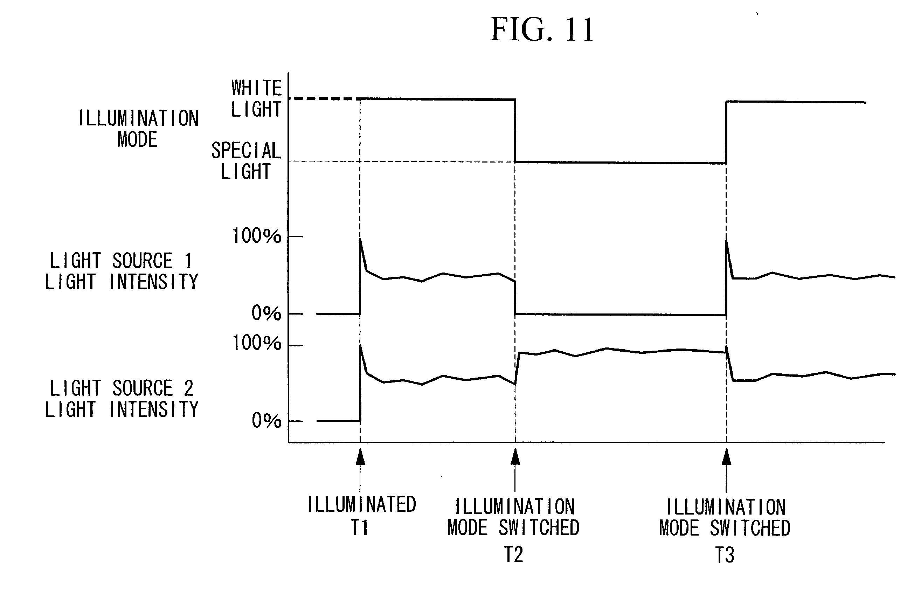

First, the case in which the illumination mode is simply switched will be described with reference to Fig. 11.
In this case, in the white light mode (between T1 and T2 and after T3), the light source 1 and the light source 2 are lit, and the intensities of the two light sources are controlled to maintain the color balance while maintaining a constant intensity ratio of the two light sources such that the brightness of the screen is constant.

In the special light mode (between T2 and T3), only the light source 2 is illuminated, and the light intensity of the light source 2 is controlled such that the brightness of the screen is constant.
As described above, by controlling the light source 1 and the light source 2, in either the white light mode or the special light mode, the brightness of the image of the subject S displayed on the image monitor 23 can be maintained constant.

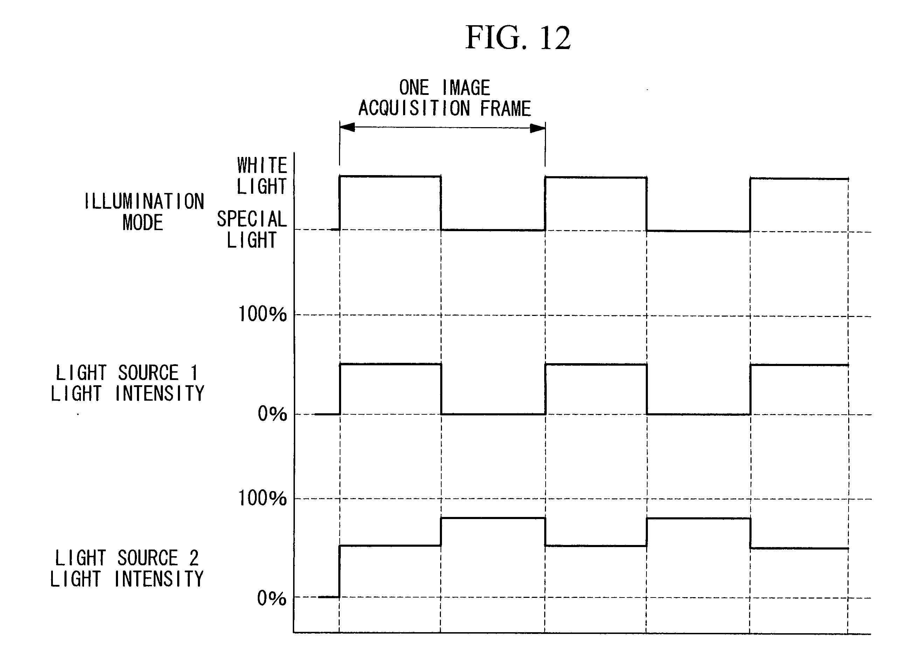

Next, a case in which two illumination modes are alternated in short periods of time will be described with reference to Fig. 12.
In such a case, when an area emitting fluorescence as a result of being excited by a specific wavelength is examined, it is difficult to determine the position of the specific area by examining only a fluorescent image. Thus, the image illuminated with the special light and a separate image examined with illumination light are alternately acquired for comparison, and the two images are displayed side by side or by superimposing them.

In the examination system 100 according to this embodiment, the illumination mode is switched every one frame (for example, 1/60 s), and the light emission intensity of the light sources is controlled such that the screen brightness in each illumination mode is constant every time the illumination mode is switched. That is, intensity control is not performed in a frame but intensity control of the light sources is performed on the basis of information about the previous image acquisition screen in the same illumination mode. In this way, the brightness of the image of the subject S displayed on the image monitor 23 can be maintained constant.

As described above, in the illumination apparatus 10 and the examination system 100 according to this embodiment, among the light L1 emitted from the light source 1, light in a predetermined band is transmitted through the dichroic mirror 5. Among the light L2 emitted from the light source 2, light in a predetermined band is transmitted through the dichroic mirror 5, while light except for that in the predetermined band is reflected in the direction of the optical axis of the light L1. In this way, the light L1 emitted from the light source 1 and the light L2 emitted from the light source 2 are combined and emitted as the combined light L5.

In this way, normal examination can be performed by, for example, lighting the light source 1 and the light source 2 and irradiating the subject S with light in a wide band (for example, white light). Moreover, special examination can be performed by, for example, illuminating only the light source 2 and irradiating the subject S with light in a narrow band (for example, blue light).
In this embodiment, it is described that, in the special light mode, the light source 1 is turned off and only the light source 2 is lit; instead, the light source 2 may be turned off and only the light source 1 may be lit.

Moreover, the dichroic mirror 5 is not only operated as light combining means for simply combining light from the two light sources in the white light mode but also is operated as a wavelength cut filter that creates a spectral characteristic required by the special light in the special light mode. In this way, it is not necessary to provide both the light combining means and the wavelength cut filter, and thus, the size and cost of the apparatus can be reduced.

By switching between the white light mode, the special light mode, etc. with the illumination-mode selecting unit 29 , the illumination state of the light sources can be controlled to irradiate the subject S with light corresponding to the examination mode. In this case, since the switching between the two illumination modes having different spectral characteristics can be performed by merely controlling the lighting of the two light sources, the illumination mode switching can be performed instantaneously, and reliability can be improved because movable parts are not included.

Moreover, by acquiring an image of the subject S with the image acquisition unit 21 and controlling the light intensities of the light sources such that the brightness of the image is constant, the light intensity of the light emitted onto the subject S is controlled in response to the intensity of the light reflected at the subject S, and the examination precision of the subject S can be improved. Also, since the illumination light intensity of the light source unit 11 is controlled such that the brightness of the subject S acquired by the image acquisition unit 21 is constant, appropriate screen brightness can be maintained even when the illumination mode is switched.

### Second Embodiment

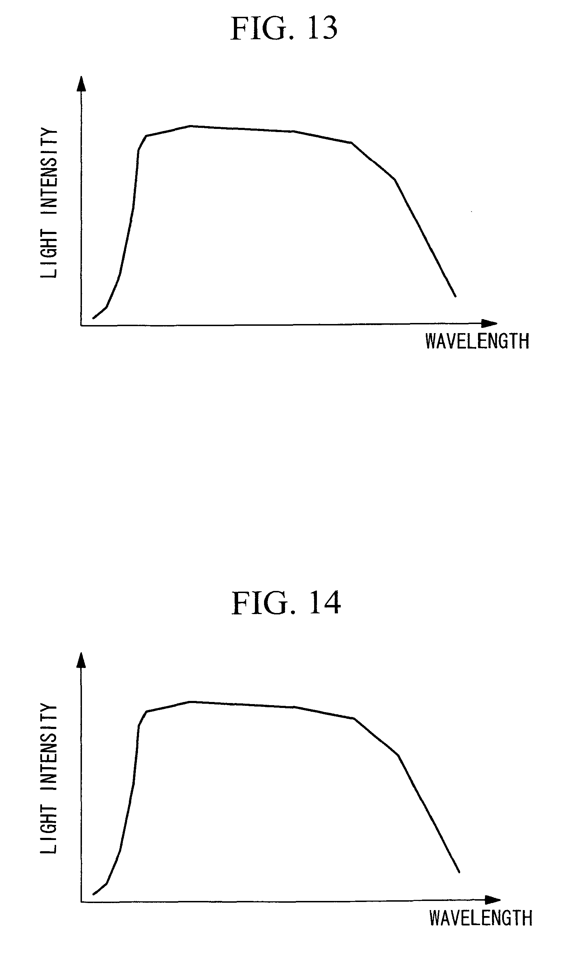

Next, an illumination apparatus according to a second embodiment of the present invention will be described with reference to Figs. 13 to 18.
The illumination apparatus according to this embodiment differs from that of the first embodiment in that the spectral characteristics of the two light sources are substantially the same. In the following, descriptions of the commonalities with the first embodiment are omitted, whereas differences are mainly described.

As illustrated in Fig. 13, a light source 1 emits light L1 having a wide wavelength band.
As illustrated in Fig. 14, a light source 2 emits light L2 having a wide wavelength band and a spectral characteristic that is substantially the same as that of the light L1.

As illustrated in Fig. 5, similar to the first embodiment, the dichroic mirror 5 has a reflection characteristic in which light in the wavelength bands between wavelengths λ1 and λ2 and greater than or equal to wavelength λ3 is transmitted, while light having a wavelength smaller than wavelength λ1 and between wavelengths λ2 and λ3 is reflected.

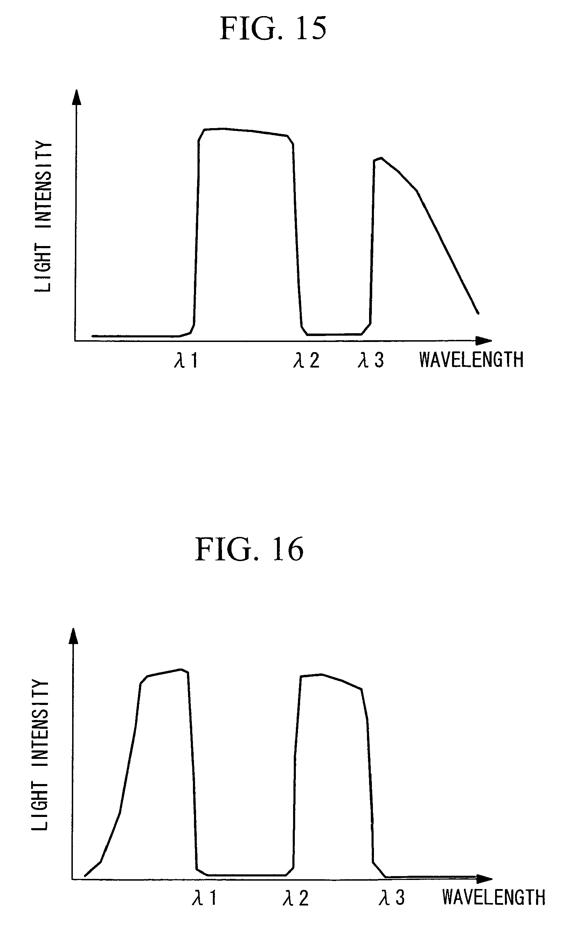

By having such a reflection characteristic, as illustrated in Fig. 15, the dichroic mirror 5 transmits light in the wavelength bands between wavelengths λ1 and λ2 and greater than or equal to wavelength λ3, among the light L1 emitted from the light source 1. Meanwhile, the dichroic mirror 5 reflects light having a wavelength smaller than wavelength λ1 and between wavelengths λ2 and λ3, among the light L1 emitted from the light source 1.

Moreover, the dichroic mirror 5 transmits light in the wavelength bands between wavelengths λ1 and λ2 and greater than or equal to wavelength λ3, among the light L2 emitted from the light source 2. Meanwhile, as illustrated in Fig. 16, the dichroic mirror 5 reflects, in the direction of the optical axis of the light L1 emitted from the light source 1, light having a wavelength smaller than wavelength λ1 and between wavelengths λ2 and λ3, among the light L2 emitted from the light source 2.

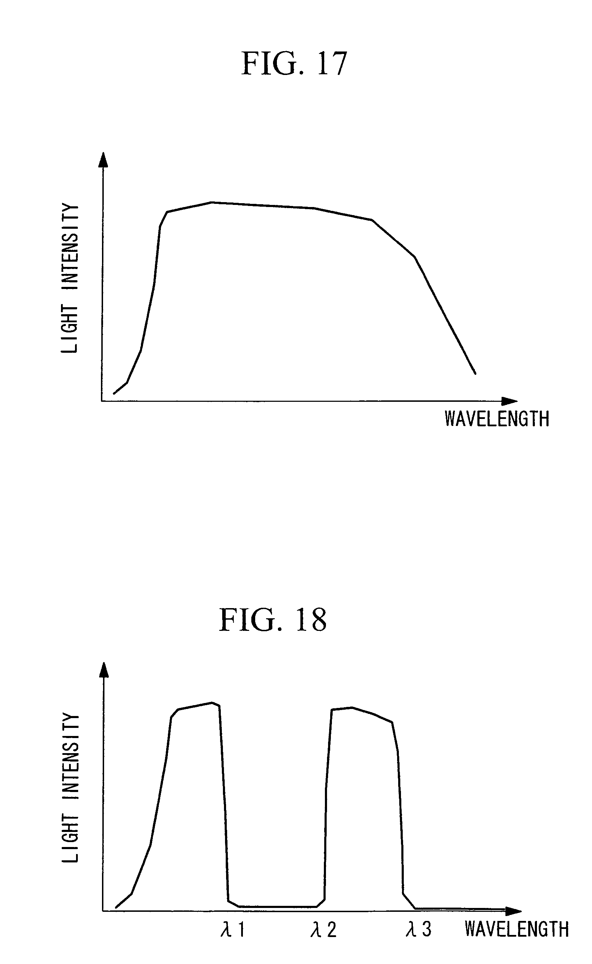

Now, in a white light mode in which the subject S is irradiated with white light, the light source 1 and the light source 2 are lit.
In such a case, the dichroic mirror 5 combines the light in the wavelength bands between wavelengths λ1 and λ2 and greater than or equal to wavelength λ3, among the light L1 emitted from the light source 1, and the light below wavelength λ1 and between wavelengths λ2 and λ3, among the light L2 emitted from the light source 2, and emits this combined light L5 in the direction of the optical axis of the light L1. As illustrated in Fig. 17, the combined light L5 has a spectral characteristic resulting from superimposing light with the spectral characteristic illustrated in Fig. 15 (the light that is transmitted through the dichroic mirror 5 among the light L1 emitted from the light source 1) and light with the spectral characteristic illustrated in Fig. 16 (the light reflected at the dichroic mirror 5 among the light L2 emitted from the light source 2).

In a special light mode in which the subject S is irradiated with special light, the light source 1 is turned off and only the light source 2 is lit.
In such a case, as illustrated in Fig. 16, the dichroic mirror 5 transmits light in wavelength bands between wavelengths λ1 and λ2 and greater than or equal to wavelength λ3, among the light L2 emitted from the light source 2, while reflecting and emitting light having a wavelength smaller than wavelength λ1 and between wavelengths λ2 and λ3 in the direction of the optical axis of the light L1 emitted from the light source 1. In such a case, the spectral characteristic of the light emitted in the direction of the optical axis of the light L1 is as illustrated in Fig. 18.

With the illumination apparatus according to this embodiment, in addition to the advantages achieved by the first embodiment described above, the subject S can be illuminated in a white light mode and a special light mode using the same light source as two light sources. Moreover, since the same light source is used, production and quality control are simplified.
In this embodiment, it is described that, in the special light mode, the light source 1 is turned off and only the light source 2 is illuminated; instead, the light source 2 may be turned off and only the light source 1 may be illuminated.

### Third Embodiment

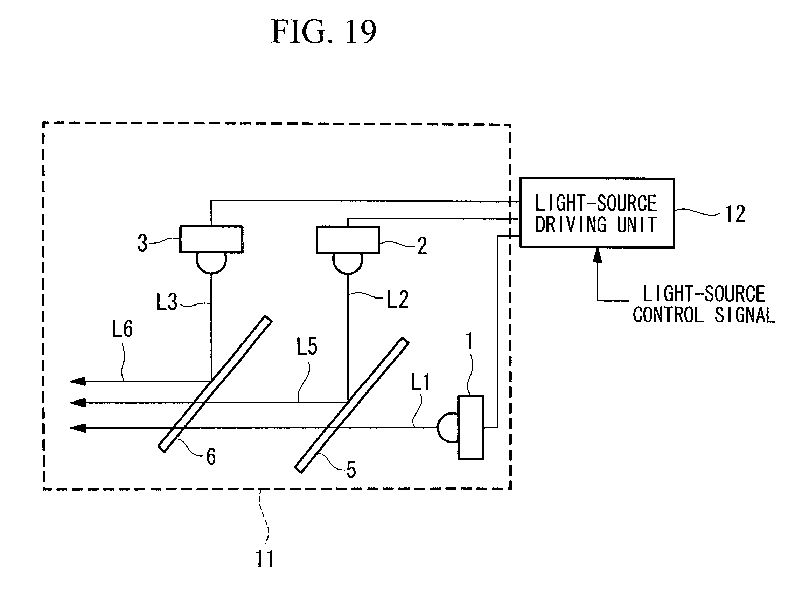

Next, an illumination apparatus according to a third embodiment of the present invention will be described mainly with reference to Figs. 19 to 24.
The illumination apparatus according to this embodiment differs from that of the embodiments described above in that three light sources and two light combining units are provided. For the illumination apparatus according to this embodiment, described below, descriptions of the commonalities with the embodiments described above are omitted, and mainly the differences are described.

As illustrated in Fig. 19, a light source unit 11 includes a light source (first light source) 1, a light source (second light source) 2, and a light source (third light source) 3, which are disposed such that optical axes thereof are orthogonal to each other; a first dichroic mirror (light combining unit) 5 disposed at the intersection of the optical axis of the light source 1 and the optical axis of the light source 2; and a second dichroic mirror (light combining unit) 6 disposed at the intersection of the optical axis of the light source 1 and the optical axis of the light source 3.

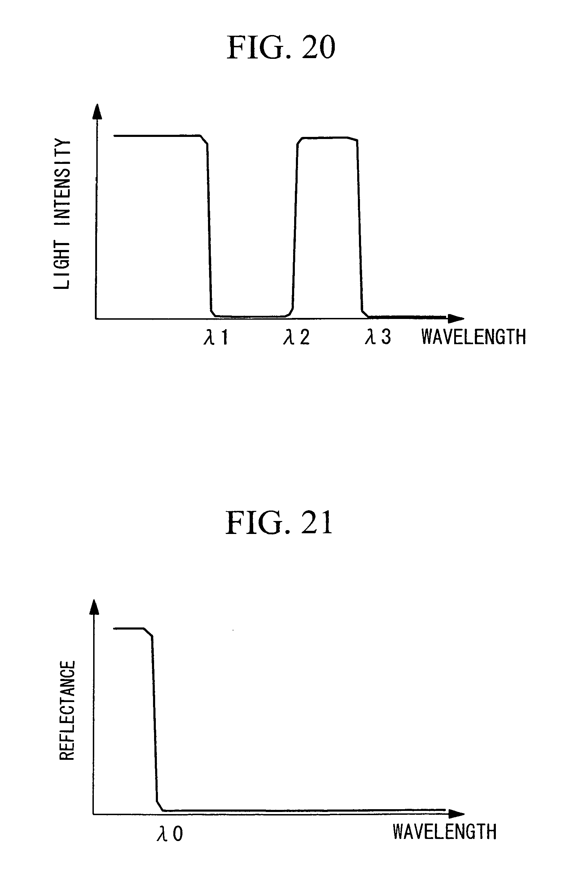

Similar to the second embodiment, the light sources 1, 2, and 3 respectively emit light L1, L2, and L3 in a wide wavelength band, as illustrated in Fig. 13.
As illustrated in Fig. 20, similar to the first embodiment, the first dichroic mirror 5 has a reflection characteristic in which light in the wavelength bands between wavelengths λ1 and λ2 and greater than or equal to wavelength λ3 is transmitted, while light having a wavelength smaller than wavelength λ1 and between wavelengths λ2 and λ3 is reflected.
As illustrated in Fig. 21, the second dichroic mirror 6 has a reflection characteristic in which light in the wavelength bands greater than or equal to wavelength λ0 is transmitted, while light having a wavelength smaller than wavelength λ0 is reflected.

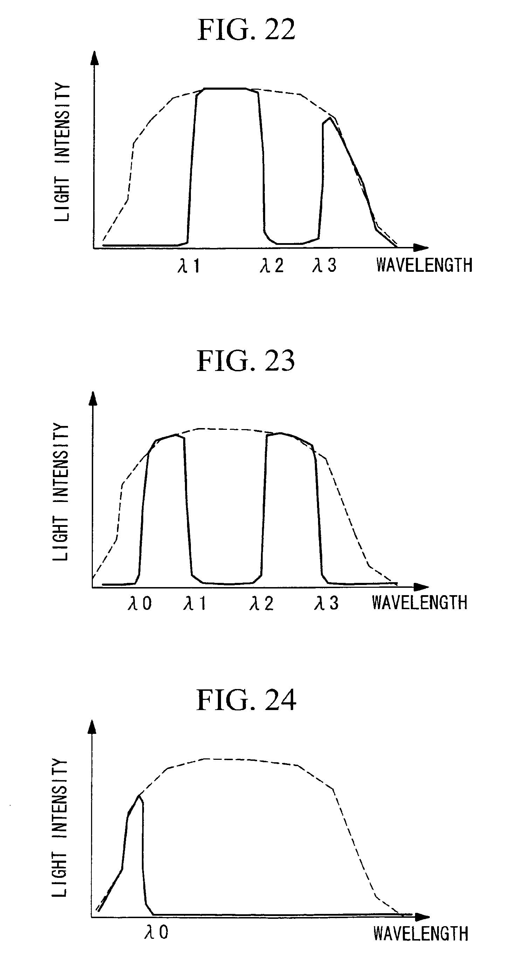

By having such a reflection characteristic, as illustrated in Fig. 22, the first dichroic mirror 5 transmits light in the wavelength bands between wavelengths λ1 and λ2 and greater than or equal to wavelength λ3, among the light L1 emitted from the light source 1. Meanwhile, the first dichroic mirror 5 reflects light having a wavelength smaller than wavelength λ1 and between wavelengths λ2 and λ3, among the light L1 emitted from the light source 1.

Moreover, the first dichroic mirror 5 transmits light in the wavelength bands between wavelengths λ1 and λ2 and greater than or equal to wavelength λ3, among the light L2 emitted from the light source 2. Meanwhile, as illustrated in Fig. 23, the first dichroic mirror 5 reflects, in the direction of the optical axis of the light L1 emitted from the light source 1, light having a wavelength smaller than wavelength λ1 and between wavelengths λ2 and λ3, among the light L2 emitted from the light source 2.

Moreover, the second dichroic mirror 6 transmits light in the wavelength band greater than or equal to wavelength λ0, among the light L3 emitted from the light source 3. Meanwhile, as illustrated in Fig. 24, the second dichroic mirror 6 reflects, in the direction of the optical axis of the light L1 emitted from the light source 1, light having a wavelength smaller than wavelength λ0, among the light L3 emitted from the light source 3.

Here, in a white light mode in which a subject S is irradiated with white light, all light sources 1, 2, and 3 are lit.
In such a case, the first dichroic mirror 5 combines the light in the wavelength bands between wavelengths λ1 and λ2 and greater than or equal to wavelength λ3, among the light L1 emitted from the light source 1, and the light below wavelength λ1 and between wavelengths λ2 and λ3, among the light L2 emitted from the light source 2, and emits this combined light L5 in the direction of the optical axis of the light L1.

The second dichroic mirror 6 combines the combined light L5 from the first dichroic mirror 5 and light in the wavelength band smaller than wavelength λ0 among the light L3 emitted from the light source 3 and emits this combined line L6 in the direction of the optical axis of the light L1. The combined light L6 has a spectral characteristic resulting from superimposing light with the spectral characteristic illustrated in Fig. 22 (the light that is transmitted through the first dichroic mirror 5 among the light L1 emitted from the light source 1), light with the spectral characteristic illustrated in Fig. 23 (the light reflected at the first dichroic mirror 5 among the light L2 emitted from the light source 2), and light with the spectral characteristic illustrated in Fig. 24 (the light reflected at the second dichroic mirror 6 among the light L3 emitted from the light source 3).

In a special light mode in which the subject S is irradiated with special light, at least one of the light sources 1, 2, and 3 is lit to radiate illumination light corresponding to the subject S. Alternatively, in the special light mode, the ratio of the light emission intensities of the light sources may be made to differ according to the subject S.

With the illumination apparatus according to this embodiment, in addition to the advantages achieved by the embodiments described above, seven different illumination modes can be provided by the ON/OFF combinations of the light sources since three light sources are provided, and various subjects S can be examined under appropriate illumination conditions. Moreover, by changing the ratio of the light emission intensities of the light sources, the examination precision of the subject S can be further improved.

### Fourth Embodiment

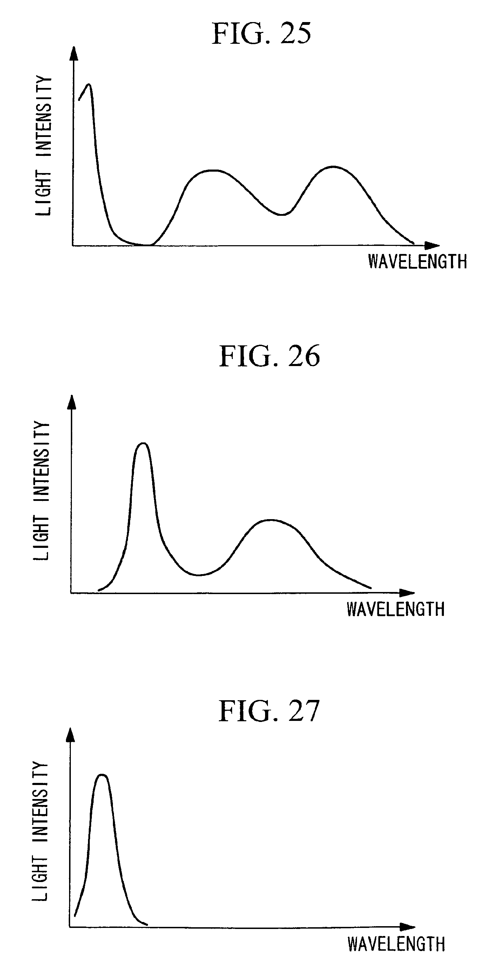

Next, an illumination apparatus according to a fourth embodiment of the present invention will be described mainly with reference to Figs. 25 to 30.
The illumination apparatus according to this embodiment has the same apparatus configuration as the third embodiment described above but differs from that of the third embodiment in that the spectral characteristics of the light sources differ. For the illumination apparatus according to this embodiment, described below, descriptions of the commonalities with the third embodiment are omitted, and mainly the differences are described.

A light source 1 is, for example, an ultraviolet LED that excites green and red fluorescers and has a spectral characteristic illustrated in Fig. 25.
A light source 2 is, for example, a blue LED that excites a yellow (YAG) fluorescer and has a spectral characteristic illustrated in Fig. 26.
A light source 3 is, for example, a purple LED that has a spectral characteristic illustrated in Fig. 27.

With the illumination apparatus having the above-described configuration, the light sources 1, 2, and 3 are all lit in the white light mode in which the subject S is irradiated with white light.
In such a case, the first dichroic mirror 5 combines the light in the wavelength bands between wavelengths λ1 and λ2 and greater than or equal to wavelength λ3, among the light L1 emitted from the light source 1, and light below wavelength λ1 and between wavelengths λ2 and λ3, among the light L2 emitted from the light source 2, and emits this combined light L5 in the direction of the optical axis of the light L1.

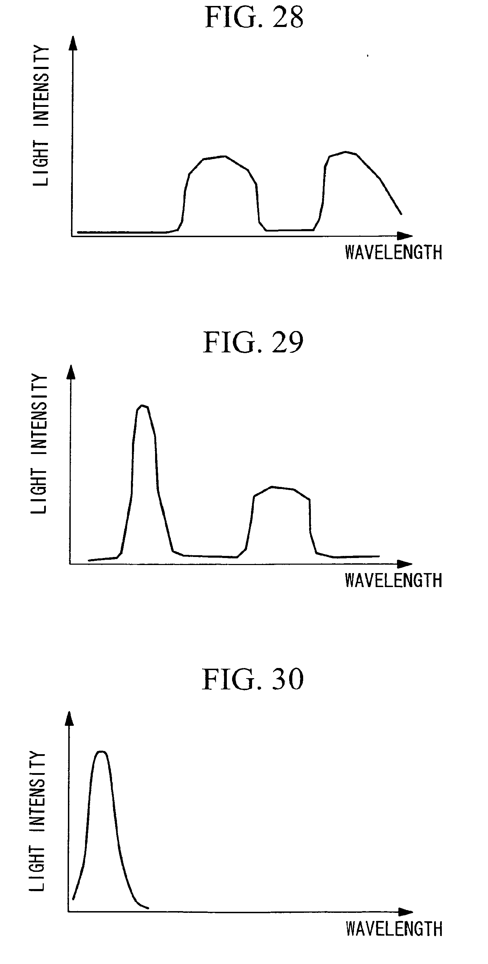

The second dichroic mirror 6 combines the combined light L5 from the first dichroic mirror 5 and light in the wavelength band smaller than wavelength λ0 among the light L3 emitted from the light source 3 and emits this combined light L6 in the direction of the optical axis of the light L1. The combined light L6 has a spectral characteristic resulting from superimposing light with the spectral characteristic illustrated in Fig. 28 (the light that is transmitted through the first dichroic mirror 5 among the light L1 emitted from the light source 1), light with the spectral characteristic illustrated in Fig. 29 (the light reflected at the first dichroic mirror 5 among the light L2 emitted from the light source 2), and light with the spectral characteristic illustrated in Fig. 30 (the light reflected at the second dichroic mirror 6 among the light L3 emitted from the light source 3).

Since the illumination apparatus according to this embodiment uses light sources that have spectral characteristics that are close to those required by the illumination modes, the light use efficiency of the light sources is improved and power consumption is reduced, compared with the third embodiment that uses a white light source.
In a special light mode in which the subject S is irradiated with special light, at least one of the light sources 1, 2, and 3 is lit. Alternatively, the ratio of the light emission intensities of the light sources may be made to differ according to the subject S.

### Fifth Embodiment

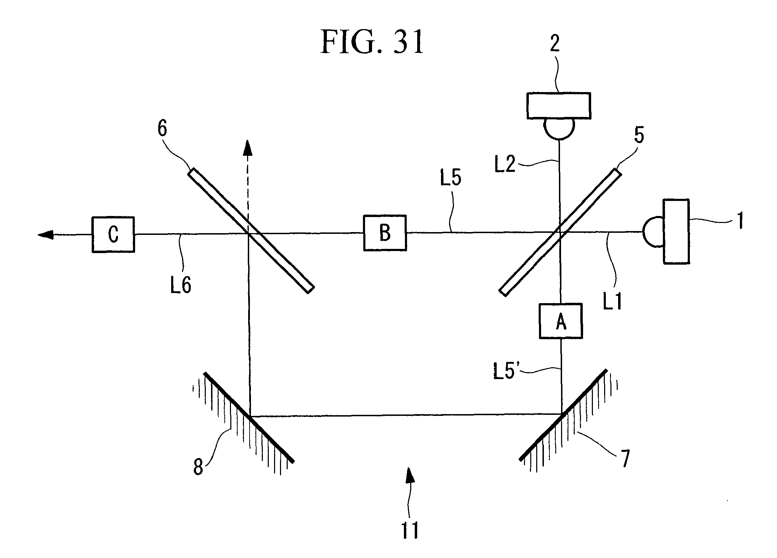

Next, an illumination apparatus according to a fifth embodiment of the present invention will be described mainly with reference to Figs. 31 to 37.
The illumination apparatus according to this embodiment differs from that of the embodiments described above in that light beams from the light sources are separated once and then recombined. For the illumination apparatus according to this embodiment, described below, descriptions of the commonalities with the embodiments described above are omitted, and mainly the differences are described.

As illustrated in Fig. 31, a light source unit 11 includes a light source (first light source) 1 and a light source (second light source) 2, which are disposed such that optical axes thereof are orthogonal to each other; a first dichroic mirror (light combining unit) 5 disposed at the intersection of the optical axis of the light source 1 and the optical axis of the light source 2; a second dichroic mirror (light combining unit) 6 disposed on the optical axis of the light source 1; a mirror 7 disposed on the optical axis of the light source 2; and a mirror 8 disposed in the reflection direction of the mirror 7.

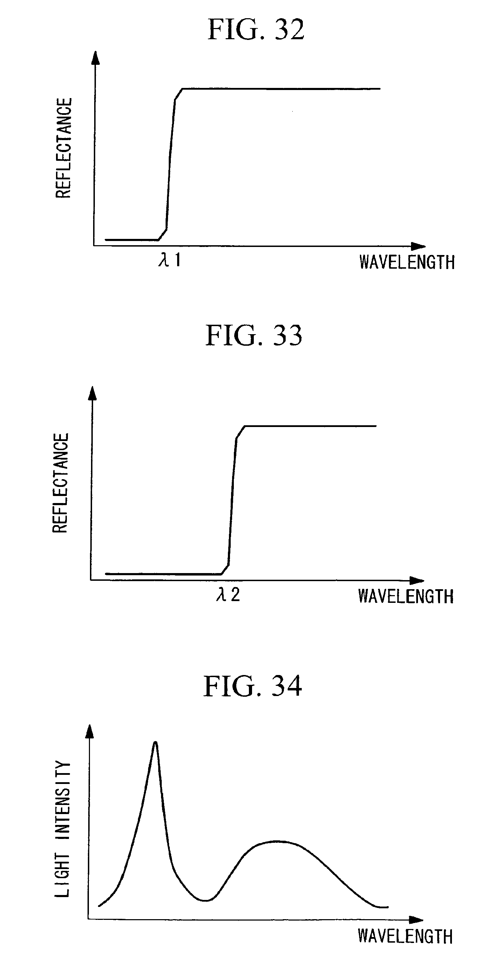

The light source 1 and the light source 2 respectively emit light L1 and light L2, which have spectral characteristics illustrated in Fig. 34.
As illustrated in Fig. 32, the first dichroic mirror 5 has a reflection characteristic in which light in the wavelength band smaller than wavelength λ1 is transmitted, while light having a wavelength greater than or equal to wavelength λ1 is reflected.
As illustrated in Fig. 33, the second dichroic mirror 6 has a reflection characteristic in which light in the wavelength band smaller than wavelength λ2 is transmitted, while light having a wavelength greater than or equal to wavelength λ2 is reflected.

With the illumination apparatus having the above-described configuration, the light source 1 and the light source 2 are lit in the white light mode in which the subject S is irradiated with white light.
In such a case, the first dichroic mirror 5 combines the light in the wavelength band smaller than wavelength λ1, among the light L1 emitted from the light source 1, and the light having a wavelength greater than or equal to wavelength λ1, among the light L2 emitted from the light source 2, and emits this combined light L5 in the direction of the optical axis of the light L1.

The first dichroic mirror 5 combines the light in the wavelength band greater than or equal to wavelength λ1, among the light L1 emitted from the light source 1, and the light having a wavelength smaller than wavelength λ1, among the light L2 emitted from the light source 2, and emits this combined light L5' toward the mirror 7.
The combined light L5' is reflected by the mirror 7 toward the mirror 8 and is reflected at the mirror 8 toward the second dichroic mirror 6.

The second dichroic mirror 6 combines the light in the wavelength band smaller than wavelength λ2, among the light L5 from the first dichroic mirror 5, and the light in the wavelength band greater than or equal to wavelength λ2, among the light L5' from the mirror 8, and emits this combined light L6 in the direction of the optical axis of the light source 1.
Accordingly, the spectral characteristics of light travelling along optical paths A, B, and C, which are illustrated in Fig. 31, form the spectral characteristic illustrated in Fig. 34.

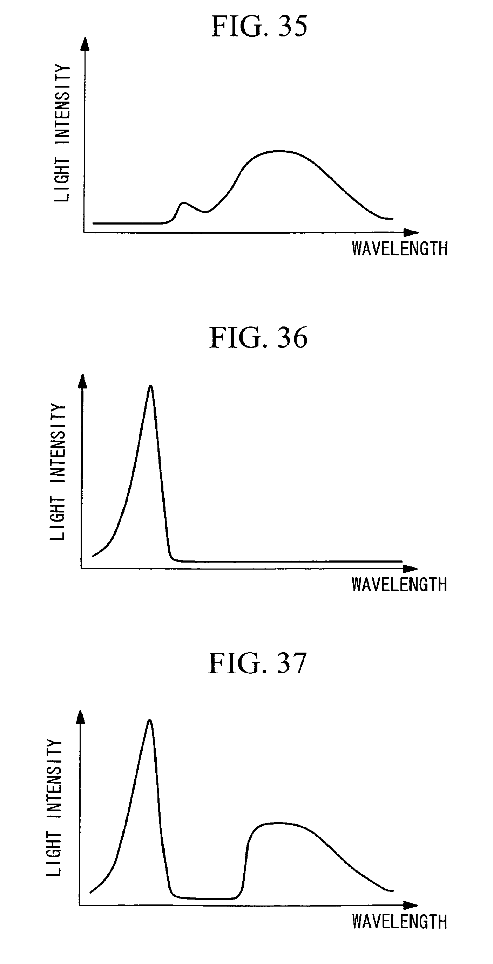

In a special light mode in which the subject S is irradiated with special light, for example, the light source 2 is turned off, and only the light source 1 is lit.
In such a case, the combined light L5' travelling along the optical path A is light in the wavelength band greater than or equal to wavelength λ1, among the light L1 emitted from the light source 1, as illustrated in Fig. 35.
The combined light L5 travelling along the optical path B is light in the wavelength band smaller than wavelength λ1, among the light L1 emitted from the light source 1, as illustrated in Fig. 36.

The combined light L6 travelling along the optical path C is a combined beam of the light in the wavelength band smaller than wavelength λ2, among the light L5 transmitted among the optical path B, and light in the wavelength band greater than or equal to wavelength λ2, among the light L5' transmitted along the optical path A, as illustrated in Fig. 37.

With the illumination apparatus according to this embodiment, in the special light mode, it is possible to produce a spectral characteristic with the required narrowband light, that is, to remove components in an unwanted wavelength band. The light that is reflected at or transmitted through the first dichroic mirror 5 can be combined by the second dichroic mirror 6, and the light usage efficiency can be improved.
In this embodiment, it is described that, in the special light mode, the light source 2 is turned off, and only the light source 1 is lit; instead, the light source 1 may be turned off and only the light source 2 may be lit.

### Sixth Embodiment

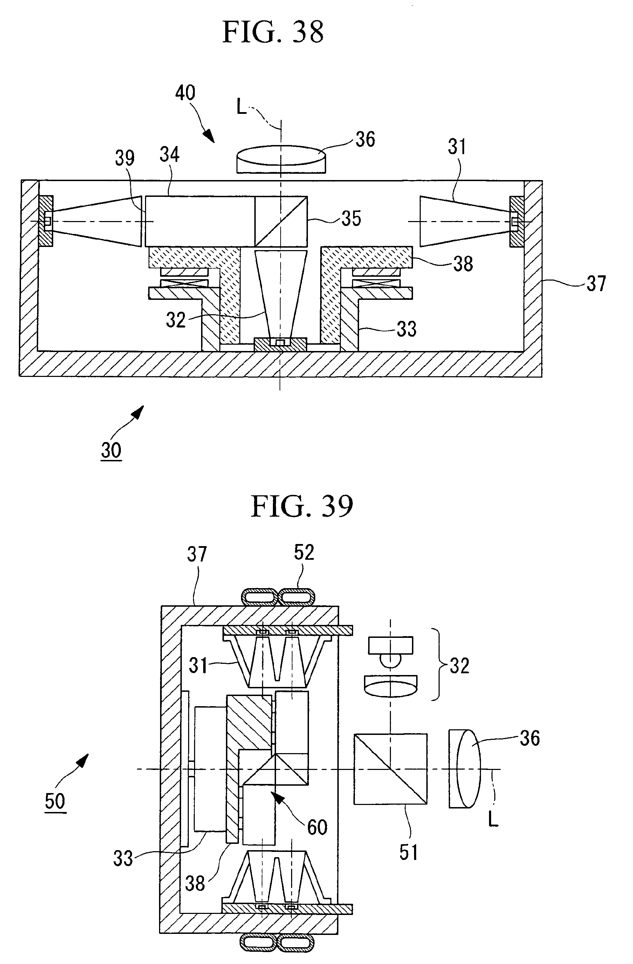

Next, an illumination apparatus according to a sixth embodiment of the present invention will be described mainly with reference to Fig. 38.
The illumination apparatus according to this embodiment differs from that of the embodiments described above in that light from a plurality of light sources arranged in a ring is superposed and emitted. For the illumination apparatus according to this embodiment, described below, descriptions of the commonalities with the third embodiment are omitted, whereas differences are mainly described.

Fig. 38 is a longitudinal sectional view showing, in outline, the configuration of an illumination apparatus 30 according to this embodiment.
As illustrated in Fig. 38, the illumination apparatus 30 includes a cylindrical base 37, a plurality of light source units (light sources) 31 arranged in a ring on the inner circumferential surface of the base 37, a light source unit (light source) 32 disposed on the center axis L of the ring, a light-guiding member 40 disposed radially inward of the light source units 31 arranged in a ring, a rod base 38 supporting the light-guiding member 40, a motor 33 rotationally driving the light-guiding member 40 and the rod base 38 as a single unit around the center axis L of the ring, and a control unit (not shown) controlling these.

The light source units 31 are disposed along the circumference at predetermined intervals with the optical axes thereof disposed radially inward of the ring and, by receiving a driving current from a power source, which is not shown, emit illumination light radially inward of the ring.

The light-guiding member 40 includes a light-guiding rod 34 disposed radially inward of the light source units 31, a dichroic prism 35 disposed radially inward of the light-guiding rod 34 on the center axis L of the ring, and an illumination lens 36 disposed in the reflection direction of the dichroic prism 35.

The light-guiding rod 34 has an incident surface 39 on which the illumination light emitted from the light source units 31 is incident and guides the illumination light incident on the incident surface 39 radially inward of the ring.

Among the illumination light guided by the light-guiding rod 34, the dichroic prism 35 reflects the light in a predetermined band in the direction of the center axis L of the ring, while transmitting the light that is outside the predetermined band. Among the illumination light emitted from the light source unit 32, the dichroic prism 35 transmits the light that is outside the predetermined band and emits it in the direction of the center axis L of the ring.

The illumination lens 36 emits the light from the dichroic prism 35 to the subject S.
By having such a configuration, the light-guiding member 40 combines the illumination light emitted radially inward from the light source units 31 and the illumination light emitted in the direction of the center axis L of the ring from the light source unit 32 and emits the combined light in the direction of the center axis L.

The rod base 38 is a cylindrical member that freely rotates the light-guiding member 40 around the center axis L with the incident surface 39 of the light-guiding member 40 disposed radially outward.
The motor 33 is disposed downstream in the direction of the center axis L of the rod base 38 and rotationally drives the light-guiding member 40 and the rod base 38 as a single unit around the center axis L.
In synchronization with the rotation of the motor 33, the control unit, which is not shown, pulsingly illuminates the light source units 31 that oppose the incident surface 39 of the light-guiding rod 34 in sequence.

The operation of the illumination apparatus 30 carrying out the above-described control will be described below.
Upon staring up the illumination apparatus 30, the light-guiding member 40 is rotationally driven by the motor 33 around the center axis L and the light source units 31 opposing the incident surface 39 of the light-guiding member 40 are pulsingly illuminated in sequence. In this way, the high-intensity illumination light emitted from the light source units 31 is sequentially incident on the incident surface 39 of the light-guiding member 40 and guided to the dichroic prism 35. Meanwhile, illumination light is also emitted from the light source unit 32 and is guided to the dichroic prism 35.

The dichroic prism 35 reflects light in a predetermined band, among the illumination light from the light source units 31 (illumination light guided by the light-guiding rod 34), in the direction of the center axis L of the ring. Among the illumination light from the light source unit 32, light that is outside the predetermined band is transmitted in the direction of the center axis L of the ring. In this way, combined light of the illumination light from the light source units 31 and the illumination light from the light source unit 32 is superposed and emitted from the illumination lens 36 to a subject to be irradiated.

With the illumination apparatus having the above-described configuration, in a white light mode in which the subject S is irradiated with white light, the light source units 31 are pulsingly illuminated in sequence, while the light source unit 32 is constantly illuminated.
In a special light mode in which the subject S is irradiated with special light, for example, the light source units 31 are pulsingly illuminated in sequence, while the light source unit 32 is turned off.

With the illumination apparatus according to this embodiment, even when the light intensity of the light emitting objects (e.g., LEDs) used for the light source units 31 during DC driving is low compared with that of the light source unit 32 (e.g., lamp light source), high-power superposed light like that matching a lamp light source can be emitted because high-current pulsingly illuminated light is superimposed and emitted.

In this embodiment, it is described that, in the special light mode, the light source units 31 are pulsingly illuminated in sequence while the light source unit 32 is turned off; instead, some of the light source units 31 may be pulsingly illuminated in sequence while the light source unit 32 is turned off, or all of the light source units 31 may be turned off while the light source unit 32 is constantly illuminated.

### Seventh Embodiment

Next, an illumination apparatus according to a seventh embodiment of the present invention will be described mainly with reference to Figs. 39 to 48.
The illumination apparatus according to this embodiment differs from that of the sixth embodiment described above in that a plurality of rings of light sources are disposed along the axial direction of the ring. For the illumination apparatus according to this embodiment, described below, descriptions of the commonalities with the sixth embodiment are omitted, and mainly the differences are described.

Fig. 39 is a longitudinal sectional diagram illustrating the schematic configuration of an illumination apparatus 50 according to this embodiment.
As illustrated in Fig. 39, the illumination apparatus 50 includes a cylindrical base 37, a plurality of light source units (light sources) 31 arranged in a ring on the inner circumferential surface of the base 37, a light-guiding member 60 disposed radially inward of the light source units 31 arranged in a ring, a rod base 38 supporting the light-guiding member 60, a motor 33 rotationally driving the light-guiding member 60 and the rod base 38 as a single unit around the center axis L of the ring, a second dichroic prism 51 disposed on the center axis L of the ring, a light source unit (light source) 32 whose optical axis is disposed toward the second dichroic prism 51, and a control unit (not shown) controlling these.

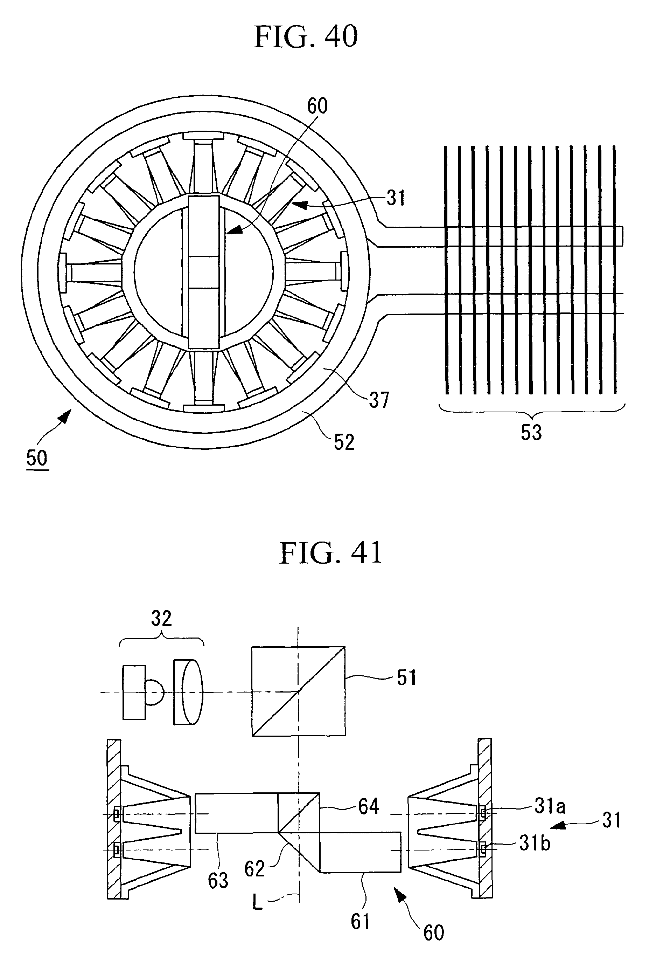

As illustrated in Fig. 40, a plurality of the light source units 31 are disposed along the circumference at predetermined intervals with the optical axes thereof disposed radially inward of the ring and, by receiving a driving current from a power source, which is not shown, emit illumination light radially inward of the ring.
As illustrated in Fig. 41, in the light source units 31, a plurality of light sources is disposed along the center axis L of the ring.

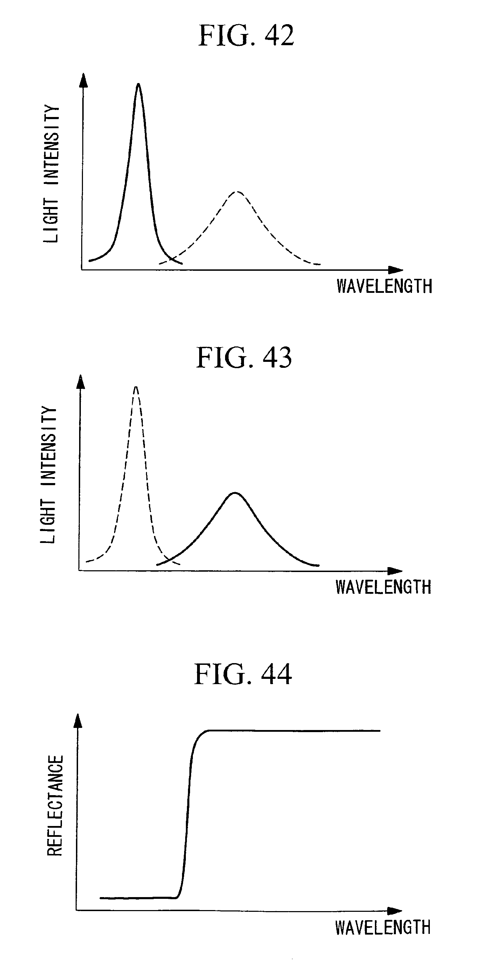

Blue LEDs 31a, having the spectral characteristic illustrated in Fig. 42, are disposed in the bottom stage of the light source units 31. Green fluorescers 31b having the spectral characteristic illustrated in Fig. 43 and excited by the blue LEDs are disposed in the top stage of the light source units 31 as light-emitting objects.

The light-guiding member 60 includes a light-guiding rod 61 disposed radially inward of the green fluorescers 31b, and a prism 62 disposed radially inward of the light-guiding rod 61 and on the center axis L of the ring, a light-guiding rod 63 disposed radially inward of the blue LEDs 31a, and a first dichroic prism 64 disposed radially inward of the light-guiding rod 63 and on the center axis L of the ring.
The light-guiding member 60 is disposed such that the directions of the incident end surfaces of the top stage and bottom stage are symmetrical with respect to the rotational axis so that the rotational balance does not become greatly off balance.

The light-guiding rod 61 guides the illumination light emitted from the green fluorescers 31b radially inward of the ring.
The prism 62 reflects the illumination light guided by the light-guiding rod 61 in the direction of the center axis L of the ring.

The light-guiding rod 63 guides the illumination light emitted from the blue LEDs 31a in the radially inward direction of the ring.
The first dichroic prism 64 has a reflection characteristic illustrated in Fig. 44 and, among the illumination light emitted from the blue LEDs 31a, reflects light in a predetermined band in the direction of the center axis L of the ring while transmitting the light that is outside the predetermined band. Among the light emitted from the green fluorescers 31b, the first dichroic prism 64 transmits the light that is outside the predetermined band and emits it in the direction of the center axis L of the ring.

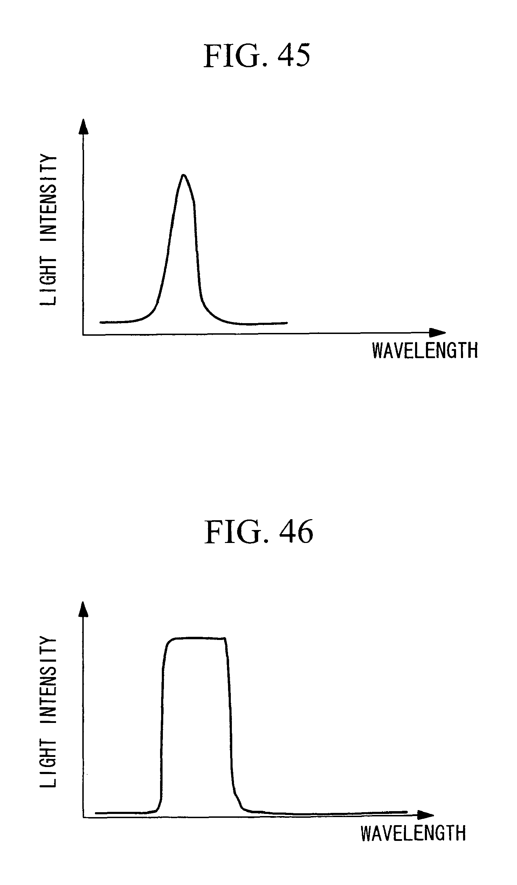

The light source unit 32 has a spectral characteristic illustrated in Fig. 45 and includes a blue-green LED as a light-emitting object.
The second dichroic prism 51 has a reflection characteristic illustrated in Fig. 46 and, among the illumination light emitted from the first dichroic prism 64, transmits light in a predetermined band in the direction of the center axis L of the ring while reflecting the light that is outside the predetermined band. Among the illumination light emitted from the light source unit 32, the second dichroic prism 51 reflects the light that is outside the predetermined band in the direction of the center axis L of the ring.

In this way, among the light emitted from the light source units 31, the second dichroic prism 51 removes unwanted light in the blue-green band, as special light, while combining the emission light from the light source unit 32 in the illumination direction. That is, the second dichroic prism 51 has the two functions of optical path combining and unwanted band removal.

As illustrated in Figs. 39 and 40, the outer circumference of the base 37 has a heat pipe 52 that collects the heat generated at the light source units 31 and transmitted to the base 37. The heat pipe 52 is also connected to a radiating fin 53 that performs heat exchange with the outside air. By having such a configuration, the heat generated at the light source units 31 is diffused outside the illumination apparatus 50 by the radiating fin 53 via the base 37 and the heat pipe 52.

The operation of the illumination apparatus 50 carrying out the above-described control will be described below.
Upon starting up the illumination apparatus 50, the light-guiding member 60 is rotationally driven around the center axis L by the motor 33 and the light source units 31 opposing the incident surface of the light-guiding member 60 are pulsingly illuminated in sequence. In this way, the high-intensity illumination light emitted from the light source units 31 is sequentially incident on the incident surface of the light-guiding member 60 and guided to the second dichroic prism 51. Meanwhile, illumination light is also emitted from the light source unit 32 and guided to the second dichroic prism 51.

Among the illumination light from the light source units 31, the second dichroic prism 51 transmits light in a predetermined band in the direction of the center axis L of the ring. Among the illumination light from the light source unit 32, light that is outside the predetermined band is reflected in the direction of the center axis L of the ring. In this way, combined light of the illumination light from the light source units 31 and the illumination light from the light source unit 32 is superposed and emitted from the illumination lens 36 to a subject to be irradiated.

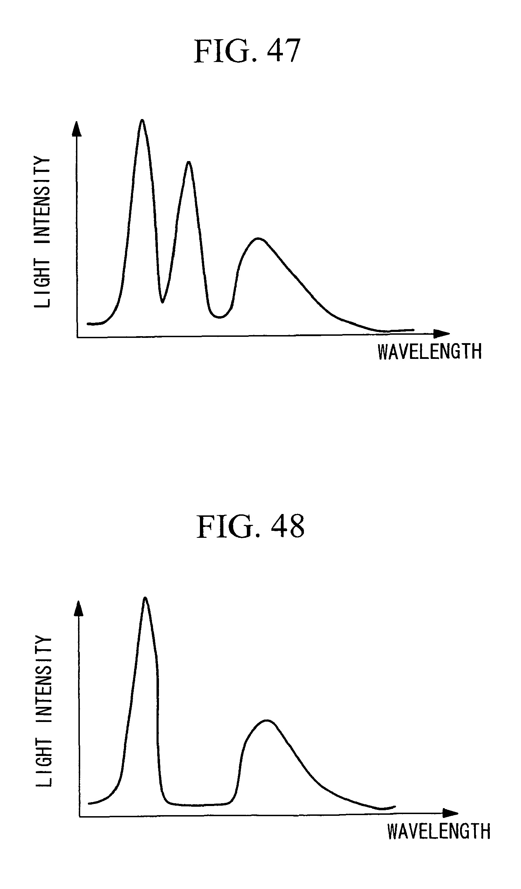

With the illumination apparatus having the above-described configuration, in a white light mode in which the subject S is irradiated with white light, the light source units 31 are pulsingly illuminated in sequence while the light source unit 32 is constantly illuminated. In this way, the subject S is irradiated with illumination light having a spectral characteristic illustrated in Fig. 47.

In a special light mode in which the subject S is irradiated with special light, for example, the light source units 31 are pulsingly illuminated in sequence while the light source unit 32 is turned off. In this way, the subject S is irradiated with illumination light having a spectral characteristic illustrated in Fig. 48.

With the illumination apparatus according to this embodiment, in addition to the advantages of the sixth embodiment, two light-emitting bands (blue/green) used in the special light mode can be constantly emitted, and the light-emitting intensity thereof can be improved.

The embodiments of the present invention have been described above in detail with reference to the drawings. The detailed configuration, however, is not limited to the embodiments, and design changes etc., may also be included so long as they do not depart from the scope of the invention. For example, in the first embodiment, an example in which the illumination apparatus according to the present invention is applied to an examination system has been described. Examples of systems to which such an illumination apparatus can be applied include an endoscope system using special light (infrared light examination or chemical fluoroscopy) or a microscope system performing fluoroscopy.

### {Reference Signs List}

- 1: light source (first light source)
- 2: light source (second light source)
- 3: light source (third light source)
- 5: dichroic mirror (light combining unit)
- 6: dichroic mirror (light combining unit)
- 10: illumination apparatus
- 11: light source unit (irradiation optical system)
- 12: light-source driving unit
- 13: illumination control unit (control unit)
- 21: image acquisition unit (light receiving unit)
- 22: image processing unit
- 23: image monitor
- 29: illumination-mode selecting unit
- 100: examination system
- L: center axis
- S: subject

## Claims

1. An illumination apparatus configured to illuminate an illumination area in a plurality of illumination modes constituted of light in different predetermined bands,
having bands in which a first band required by one illumination mode of the plurality of illumination modes is narrower than a second band required by another illumination mode,
the apparatus comprising:
a plurality of light sources configured to emit illumination light in at least the first band;
a light combining unit configured to transmit light in the first band, among the illumination light emitted from at least one light source of the plurality of light sources, toward the illumination area as transmitted light while reflecting light that is outside the first band, among the illumination light emitted from the other light sources, toward the illumination area as reflected light, and to optically combine the transmitted light and the reflected light;
an illumination-mode selecting unit configured to select one illumination mode from the plurality of illumination modes; and
a control unit configured to control the light intensity of light emitted from the plurality of light sources on the basis of the selected illumination mode,
wherein the one illumination mode is a first illumination mode for illuminating only the one light source, and the other mode is a second illumination mode for illuminating both the one light source and the other light sources.

2. The illumination apparatus according to Claim 1, wherein the one light source and the other light sources are light sources emitting white illumination light.

3. An examination system comprising:
the illumination apparatus according to Claim 1;
an irradiation optical system configured to irradiate a subject with light combined at the light combining unit; and
a light receiving unit configured to receive light reflected at the subject,
wherein the control unit controls the emission light intensity of the plurality of light sources on the basis of the intensity of the reflected light received by the light receiving unit.

4. The examination system according to Claim 3, wherein
the light receiving unit is an image acquisition element configured to acquire an image of the subject, and
the control unit controls the emission light intensity of the plurality of light sources so that the brightness of the image acquired by the image acquisition element is substantially constant.

5. An illumination apparatus configured to illuminate an illumination area in a plurality of illumination modes constituted of light in different predetermined bands,
having bands in which a first band required by one illumination mode of the plurality of illumination modes is narrower than a second band required by another illumination mode,
the apparatus comprising:
a plurality of light sources configured to emit illumination light in at least the first band;
a light combining unit configured to reflect light in the first band, among the illumination light emitted from at least one light source of the plurality of light sources, toward the illumination area as reflected light while transmitting light that is outside the first band, among the illumination light emitted from the other light sources, toward the illumination area as transmitted light, and to optically combine the transmitted light and the reflected light;
an illumination-mode selecting unit configured to select one illumination mode from the plurality of illumination modes; and
a control unit configured to control the light intensity of light emitted from the plurality of light sources on the basis of the selected illumination mode,
wherein the one illumination mode is a first mode for illuminating only the one light source, and the other illumination mode is a second illumination mode for illuminating both the one light source and the other light sources.

6. The illumination apparatus according to Claim 5, wherein the one light source and the other light sources are light sources emitting white illumination light.

7. An examination system comprising:
the illumination apparatus according to Claim 5;
an irradiation optical system configured to irradiate a subject with light combined at the light combining unit; and
a light receiving unit configured to receive light reflected at the subject,
wherein the control unit controls the emission light intensity of the plurality of light sources on the basis of the intensity of the reflected light received by the light receiving unit.

8. The examination system according to Claim 7, wherein
the light receiving unit is an image acquisition element configured to acquire an image of the subject, and
the control unit controls the emission light intensity of the plurality of light sources so that the brightness of the image acquired by the image acquisition element is substantially constant.
